# EUROPEAN PATENT SPECIFICATION

(11) **EP 4 014 903 B1**
(45) Date of publication and mention of the grant of the patent: **19.07.2023**
(21) Application number: 20214028.1
(22) Date of filing: 15.12.2020
(51) Int. Cl.: A61B 17/34, A61B 5/00, A61N 1/05, A61N 1/375, A61N 1/36

(54) **IMPLANT DELIVERY DEVICE**
VORRICHTUNG FÜR IMPLANTATEINSATZ
DISPOSITIF DE POSE D'IMPLANT

(43) Date of publication of application: 22.06.2022
(73) Proprietor: Capri Medical Limited, Dublin D04 W2K0 (IE)
(72) Inventor: Mullins, John, Dublin, D04 W2K0 (IE); Ward, Fergal, Dublin, D04 W2K0 (IE); Hegarty, Dominic, Dublin, D04 W2K0 (IE)
(74) Representative: HGF

(56) References cited:
- EP-A1- 3 616 748
- US-A1- 2011 034 939
- US-A1- 2012 323 254
- US-A1- 2017 224 986
- US-A1- 2018 256 906
- US-B2- 8 032 220

## Description

This invention is defined in the appended claims and generally relates to an implant delivery device for implanting a medical implant, such as a neuromodulation implant, in a patient.

### BACKGROUND

It is known to provide an implantable neurostimulator comprising a housing and an electrode. A power antenna, microcontroller, and communication antenna are disposed in the housing for receiving power from an external source and receiving/transmitting sensor information relating to the electrode. A delivery system can be used to position the neurostimulator in a patient, in particular proximate to a nerve, by cutting an opening in the patient and passing the delivery system into the opening to position the implantable neurostimulator.

### BRIEF SUMMARY OF THE DISCLOSURE

In accordance with the present disclosure there is provided an implant delivery device for implanting a medical implant in a patient, the medical implant comprising an implant housing and an elongate electrode lead extending from the implant housing, and wherein the implant delivery device comprises:
a handle,
a first needle fixed to the handle and extending in a longitudinal direction, the first needle comprising a lumen adapted to receive the implant housing of the medical implant, and
a second needle having a higher gauge than the first needle, the second needle comprising a lumen adapted to receive the elongate electrode lead of the medical implant, and wherein the second needle is retractably mounted to the handle such that the second needle is movable between:
   an extended position in which the second needle extends beyond the first needle in the longitudinal direction such that during use the first needle and the second needle can simultaneously position the implant housing at a first depth and the electrode lead at a second depth greater than the first depth, and
   a retracted position in which the second needle is retracted towards the handle to release the electrode lead from the second needle.

In examples, the first needle and the second needle each comprise a sharp tip, for example a bevel tip, adapted to pierce the skin and penetrate the tissue of the patient during use to position first needle and the second needle for releasing the electrode. In examples, the first needle is configured to only penetrate the skin and underlying fatty tissue during use. Accordingly, the first needle, which is larger, only penetrates the patient to the minimum required depth and reduces tissue damage.

In examples, the implant housing is arranged in the lumen of the first needle such that the implant housing can be pulled out of the lumen of the first needle. Accordingly, a holding force provided between the electrode lead and the tissue of the patient after the electrode lead has been released will pull the implant housing out of the lumen of the first needle as the implant delivery device is removed from the patient.

In examples, the implant delivery device further comprises a retaining member adapted to releasably secure the implant housing in the lumen of the first needle. The retaining member may be operable by an operator to release the implant housing and allow the implant housing to move out of the first needle and into a deployed position in the patient.

In examples, the retaining member may comprise a retaining tab formed in a wall of the first needle and adapted to engage the implant housing in the lumen of the first needle. The retaining tab may be formed by a U-shaped cut in the first needle that is partially deformed into the lumen of the first needle. The implant housing may include a recess arranged to correspond with the retaining tab. The first needle may include multiple retaining tabs distributed longitudinally and/or circumferentially about the first needle and adapted to engage the implant housing.

In examples, the implant housing may comprise an attachment point, for example a hole. The retaining member may extend from a part of the handle to the attachment point of the implant housing, and the retaining member may be detachable to release the implant housing.

In examples, the retaining member may comprise a thread that is looped through the attachment point of the implant housing, and the thread may be split by the operator, for example cut or untied, to release the implant housing. In examples, the thread may comprise a suture, for example comprising Nylon, Monocryl, Vicryl, silk, or Prolene, or a metallic wire such as steel wire, or other material such as Kevlar.

In examples, the retaining member may comprise a shape memory member having a hooked end adapted to engage the attachment point of the implant housing. An opposite end of the shape memory housing may be arranged to be moved to deform the hooked end to release the implant housing. The opposite end of the shape memory member may be attached to a sliding tab arranged to be slid by an operator to deform the hooked end.

In examples, the second needle may comprise an actuation tab arranged adjacent to the handle. The actuation tab may be configured for actuation by a user to move the second needle into the retracted position. The actuation tab may be movable in a direction away from the handle and away from the patient to move the second needle into the retracted position.

In examples, the implant delivery device may further comprise a locking mechanism adapted to lock the second needle in the extended position.

In examples, the implant delivery device comprises a locking slot in the handle. The second needle may be adapted to engage the locking slot such that in one rotational position of the second needle relative to the handle the second needle is locked in the axial direction relative to the handle, and in another rotational position of the second needle relative to the housing the second needle is movable relative to the handle in an axial direction. In examples, the locking slot may comprise an L-shaped or J-shaped slot. In examples, the handle may comprise a locking ring and the locking slot may be formed in the locking ring.

In examples, the implant delivery device may further comprise an implant deployment member comprising a pusher extending from adjacent to the handle into the lumen of the first needle. The implant deployment member may be slidable relative to the first needle to push the implant housing out of the first needle. The implant deployment member may be hollow and an implant housing retaining member may extend through the implant deployment member. The implant deployment member may include a tab for an operator to actuate the implant deployment member. An implant housing retaining member may be releasable from the tab of the implant deployment member.

In examples, the second needle may comprise a slot extending along one side and extending into the lumen. The slot may extend to a tip of the second needle. The slot may extend from the tip of the second needle into the lumen of the first needle when the second needle is in the extended position.

In examples, the first needle and the second needle are non-concentrically arranged such that a portion of the electrode lead extends from the lumen of the second needle through the slot to the implant housing during use.

In examples, the second needle is at least partly disposed in the lumen of the first needle. For example, the second needle extends alongside the implant housing within the lumen of the first needle.

In examples, the lumen of the first needle comprises a primary portion adapted to receive the implant housing and a secondary portion adapted to receive the second needle. The primary portion and the secondary portion may each comprise a partially circular cross-section. The first needle may have a tear drop cross-sectional profile.

In examples, the implant delivery device may further comprise a medical implant comprising an implant housing disposed in the lumen of the first needle and an electrode lead extending from the implant housing into the lumen of the second needle.

The combined implant delivery device and medical implant may be packaged for single use.

In examples, the medical implant is an implantable neurostimulation device. The medical implant may be configured for stimulating a nerve of the patient, for example the greater occipital nerve. US 2018/256906 and EP 3 616 748 describe different implant delivery devices.

### BRIEF DESCRIPTION OF THE DRAWINGS

Embodiments of the invention are further described hereinafter with reference to the accompanying drawings, in which:
FIG. 1 shows a medical implant;
FIG. 2 show the medical implant in position in a patient, particularly in a subcutaneous position;
FIG. 3 shows a schematic cross-section of a part of a delivery device for implanting the medical implant into the patient, particularly a first needle and a second needle of the delivery device;
FIG. 4 shows a schematic cross-section of the delivery device, including a handle, in a configuration before implanting the medical implant;
FIG. 5 shows a schematic cross-section of the delivery device after the electrode lead of the medical implant has been released;
FIG. 6 shows a schematic cross-section of the delivery device after the medical implant has been released;
FIG. 7 shows a perspective view of an end of the first needle and the second needle, and a medical implant retained by the first and second needles;
FIG. 8A shows the second needle;
FIG. 8B shows the first needle;
FIG. 9 shows an example delivery device for implanting the medical implant into the patient;
FIG. 10 shows the second needle of the delivery device of FIG. 9, with an actuation tab;
FIG. 11 shows an alternative view of the delivery device of FIG. 9;
FIG. 12 shows a locking mechanism for the second needle and actuation tab of the delivery device of FIG. 9;
FIGS. 13A and 13B show an example of a retaining mechanism for releasably retaining the implant housing of the medical implant in the first needle;
FIGS. 14A to 14C show a further example of a retaining mechanism for releasably retaining the implant housing of the medical implant in the first needle;
FIGS. 15A to 15C show a further example of a retaining mechanism for releasably retaining the implant housing of the medical implant in the first needle; and
FIGS. 16A to 16D illustrate operation of the delivery device to implant the medical implant in a patient;

### DETAILED DESCRIPTION

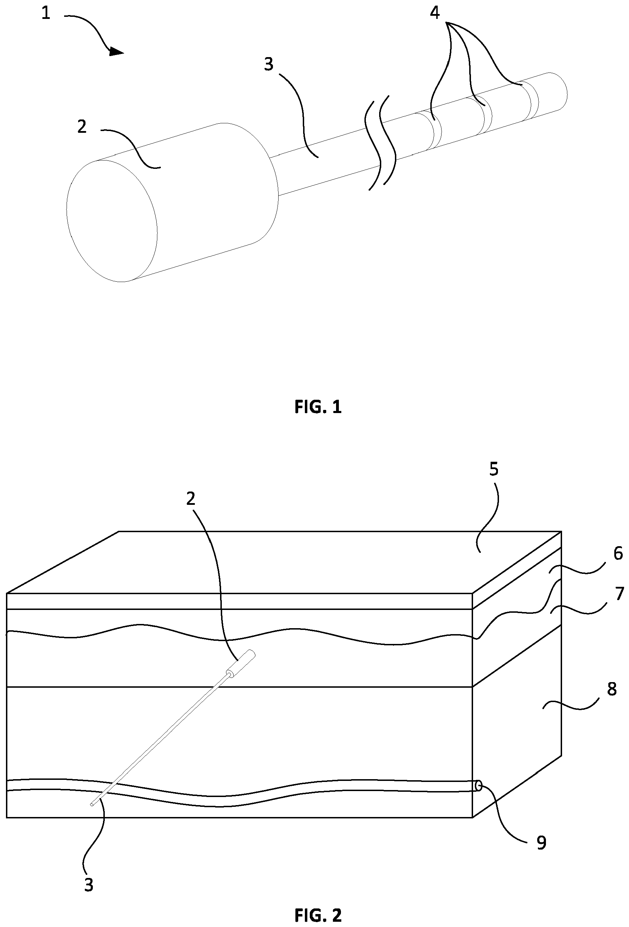

FIGS. 1 and 2 illustrate the medical implant 1 and use of the medical implant 1 in a patient. As described further hereinafter, the medical implant 1 is implantable in a patient and operable to sense and/or stimulate a nerve. In some examples, the medical implant 1 is implantable to sense and/or stimulate the greater occipital nerve, although the same or similar implant may be implantable to sense and/or stimulate other nerves, particularly other peripheral nerves of the peripheral nervous system. In examples, the medical implant 1 may be implantable to sense and/or stimulate the tibial nerve, the sacral nerve (e.g., to treat urinary incontinence) or the vagus nerve (e.g., to regulate pancreatic secretion).

As shown in FIG. 1, the medical implant 1 comprises an implant housing 2 and an elongate electrode lead 3. The implant housing 2 may house electronics components of the medical implant 1, including for example a printed circuit board, a receiver/transmitter, a wireless power receiver, and/or sensor electronics. In examples, the implant housing is hermetically sealed.

The electrode lead 3 extends from the implant housing 2 and is flexible. The electrode lead 3 includes at least one electrode 4, in some examples multiple electrodes 4 spaced along the length of the electrode lead 3. The electrodes 4 are connected to the electronics within the implant housing 2.

In examples, the implant housing 2 may have a diameter of between about 0.5 millimetres and about 5 millimetres, for example between about 1 millimetre and about 3 millimetres. The implant housing 2 may have a length of up to about 10 millimetres, for example up to about 5 millimetres. In examples, the electrode lead 3 may have a diameter of between about 0.3 millimetres to about 1.5 millimetres, for example between about 0.5 millimetres and 1.3 millimetres. The electrode lead 3 may have a length of up to about 100 millimetres, for example up to about 50 millimetres, for example about 50 millimetres. However, it will be appreciated that the dimensions of the implant housing 2 would correspond to the size of the electronics housed within the implant housing 2, and the length of the electrode lead 3 would correspond to the anatomy surrounding the targeted nerve, so a shorter or longer electrode lead 3 may be appropriate depending on the depth of the nerve within the muscle tissue.

As shown in FIG. 2, once implanted in a patient the medical implant 1 is positioned below the surface of the skin, in particular below the epidermis 5. The implant housing 2 may be positioned in the dermis 6 or in the subcutaneous tissue 7. Positioning the implant housing 2 in the subcutaneous tissue 7 may be beneficial to cause less damage and/or irritation to the patient.

As illustrated, the electrode lead 3 extends from the implant housing 2, through the underlying tissue, in particular muscle 8, to a position proximal to the target nerve 9. The electrode lead 3 is positioned such that the electrodes (4, see FIG. 1) are in contact with or proximal to the nerve 9, and so the electrodes 4 can be used to sense and/or stimulate the nerve 9.

An external device (not shown) can wirelessly power, and wirelessly communicate with, the medical implant 1, in particular the electronics in the implant housing 2. The external device may be positioned on the skin proximal to the medical implant 1. The external device may be adhered to the skin proximal to the medical implant 1.

In examples, the medical implant 1 is a neurostimulation implant that is implanted to target the greater occipital nerve of a patient, and the electrodes 4 provided with an electrical signal, such as a current, to stimulate the greater occipital nerve. In examples, the electrical signal may be a voltage regulated stimulation. Such stimulation can provide relief for chronic pain, for example occipital neuralgia, intractable migraine, and/or other therapeutic benefits.

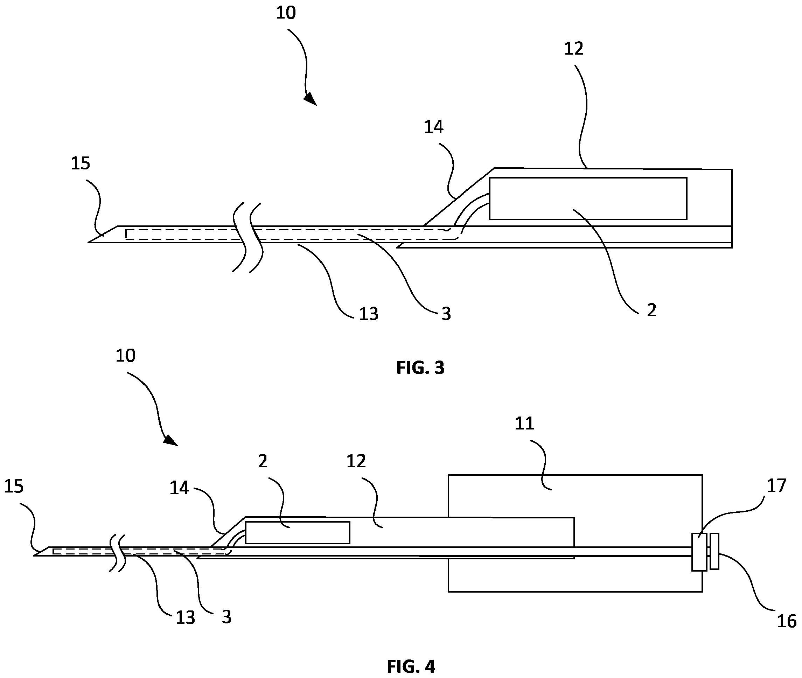

FIGS. 3 to 16D illustrate examples of an implant delivery device 10 for implanting the medical implant 1 illustrated in FIG. 1 in the location illustrated in FIG. 2.

As shown in FIG. 3, the implant delivery device 10 includes a first needle 12 and a second needle 13. The first and second needles 12,13 are parallel and both extend in a longitudinal direction. The second needle 13 extends further than the first needle 12. In particular, the first needle 12 has a tip 14, such as a bevel tip, and the second needle 13 extends past the tip 14 of the second needle. The second needle 13 also has a tip 15, in particular a bevel tip. The second needle 13 has a higher gauge than the first needle 12, and in particular the second needle 13 has a smaller diameter than the first needle 12.

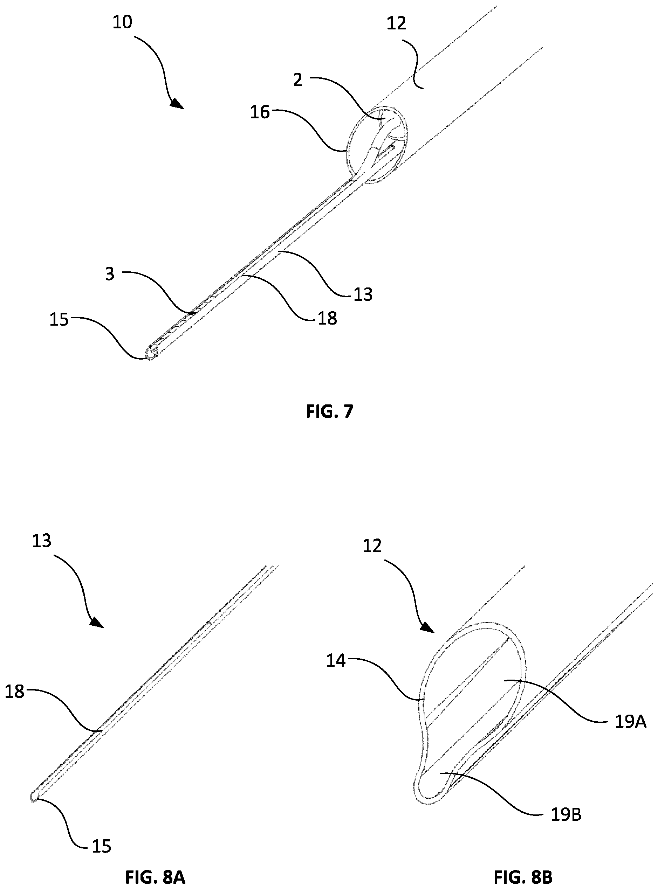

During use, the implant housing 2 is received in the first needle 12, in particular in a lumen of the first needle 12. During use, the electrode lead 3 is received in the second needle 13, in particular in a lumen of the second needle 13. The electrode lead 3 extends along a substantial part of the second needle 13 towards the tip 15, as illustrated. A part of the electrode lead 3 adjacent to the implant housing 2 extends through an opening in the second needle 13, as described further with reference to FIG. 7. Accordingly, during use the medical implant 1 is housed within the first and second needles 12, 13 of the delivery device 10.

As shown in FIG. 3, the first needle 12 and the second needle 13 are axially offset. In particular, a central axis of the first needle 12 is offset from a central axis of the second needle 13. In the illustrated example the second needle 13 extends into the first needle 12 (in particular into the lumen of the first needle), such that the second needle 13 is partly accommodated within the first needle 12 alongside the implant housing 2.

Referring to FIG. 7, the second needle 13 includes a slot 18 to permit a part of the electrode lead 3 to extend out of the second needle 13 and connect to the implant housing 2, as shown in FIG. 3. The slot 18 may extend to the tip 15 of the second needle 13, as described further hereinafter.

In some examples the second needle 13 may comprise a sheath, having an opening extending entirely or substantially along one side.

As described in further detail hereinafter, during use the first needle 12 and the second needle 13 both penetrate the patient's skin to simultaneously position the implant housing 2 at a first depth and the electrode lead 3 at a second depth within the patient. The implant delivery device 10 then releases and deploys the medical implant 1 to leave the medical implant 1 in the position illustrated in FIG. 2. The tips 14, 15 of the first and second needles 12, 13, respectively, are sharp tips adapted to pierce the patient's skin and penetrate the tissue to position the first and second needles 12, 13 at the appropriate depth. The tips 14, 15 may be bevel tips, as would be known to the skilled person.

In examples, the first needle 12 may have a gauge of between 6 gauge and 15 gauge, for example 10 gauge. In examples, the second needle 13 may have gauge of between 15 gauge and 25 gauge, for example 20 gauge.

In various examples described hereinafter, the second needle 13 is retractable relative to the first needle 12, to deploy the electrode lead 3. The slot 18 (see FIG. 7) along the second needle 13 permits deployment of the electrode lead 3.

In examples, the implant housing 2 is releasably attached to the first needle 12 (or another part of the implant delivery device 10) and is released prior to deployment.

In examples, the implant housing 2 may be deployed from the first needle 12 simply by pulling the implant delivery device away from the patient and relying on friction between the electrode lead 3 and the patient's tissue to hold the medical implant in place and pull the implant housing 2 from the first needle 12. In other examples, the implant delivery device 10 may include a deployment member adapted to push the implant housing 2 out of the first needle 12 to deploy the implant housing 2 at the appropriate anatomical site.

FIGS. 4 to 6 show additional parts of the implant delivery device 10 and also illustrate operation of the implant delivery device 10 to deploy the medical implant 1 into the patient.

As illustrated, the implant delivery device 10 further includes a handle 11. The handle 11 is adapted to be held by an operator. The first needle 12 is fixed to the handle 11.

The second needle 13 extends through the first needle 12 and through the handle 11. An actuation tab 16 is provided on an end of the second needle 13 opposite to the tip 15. In particular, the actuation tab 16 may be a gripping handle or similar for the operator to grip.

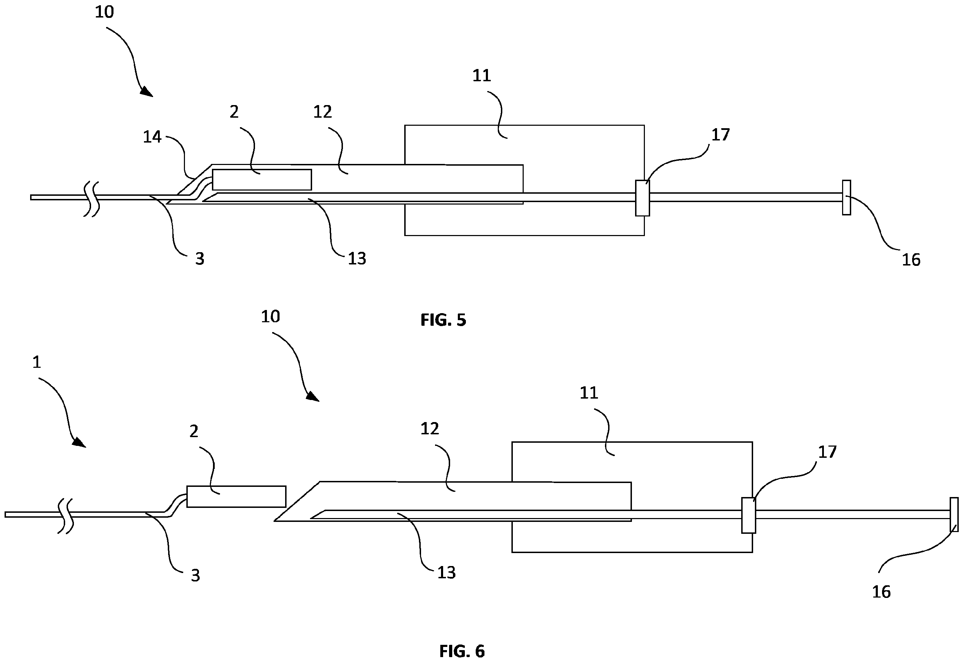

The second needle 13 is retractable relative to the first needle 12. In particular, the second needle 13 can slide through the first needle 12 and handle 11, from the position shown in FIG. 4 and the position shown in FIG. 5. Retracting the second needle 13 in this way deploys the electrode lead 3. The slot 18 (see FIG. 7) along the second needle 13 provides for the part of the electrode lead 3 that connects to the implant housing 2. The slot 18 extends to the tip 15 of the second needle 13.

A locking device 17 is provided to lock the second needle 13 to the handle 11 and/or first needle 12. As shown, the locking device 17 may be provided at or near the actuation tab 16, and in examples locks the actuation tab 16 to the handle 11. The locking device 17 locks the second needle 13 in the extended position shown in FIG. 4.

In the position shown in FIG. 4 the operator can insert the implant delivery device 10 into the patient. The position of the second needle 13 is locked relative to the handle 11, so the operator can push the implant delivery device 10 into the patient by the handle 11.

Referring to FIGS. 4 and 2, as the implant delivery device 10 is pushed into the patient the second needle 13 first penetrates the skin 5, and as the implant delivery device 10 is pushed further the first needle 12 then penetrates the skin 5. The first and second needles 12, 13 are thereby positioned at the appropriate depths in the patient.

In examples, the operator may use an ultrasound imaging device to monitor the positions of the first needle, and in particular the second needle 13, to guide the second needle 13 towards the target nerve 9.

Once the implant delivery device 10 is in position, with the tip 15 of the second needle 13 (and the electrode lead 3 within the second needle 13) being positioned adjacent to the nerve, and the tip 14 of the first needle 12 (and the implant housing 2 within the first needle 12) being positioned in the subcutaneous tissue, the second needle 13 can be retracted to the position shown in FIG. 5.

The second needle 13 is retracted by unlocking the locking mechanism 17 and pulling on the actuation tab 16 relative to the handle 11 to slide the second needle 13 to the retracted position shown in FIG. 5. During retraction of the second needle 13 the handle 11 and first needle 12 remain stationary. As shown, once the second needle 13 is retracted the electrode lead 3 is deployed and exposed to the surrounding tissue (and nerve).

Next, as shown in FIG. 6, the implant delivery device 10 is withdrawn from the patient and the implant housing 2 is deployed from the first needle 12. In this example the friction between the electrode lead 3 and the tissue (see muscle 8 in FIG. 2) is enough to hold the medical implant 1 in place as the implant delivery device 10 is withdrawn, thereby deploying the implant housing 2 out of the first needle 12 as the implant delivery device 10 is withdrawn from the patient.

In other examples detailed hereinafter the implant delivery device 10 may include a deployment member configured to push the implant housing 2 out of the first needle 12. In some examples detailed hereinafter, the implant delivery device 10 may have a retaining member arranged to releasably attach the implant housing 2 to the first needle 12 and/or handle 11, and the retaining member can release the implant housing 2 after the second needle 13 is retracted and before the first needle 12 is removed from the patient.

FIGS. 7, 8A and 8B illustrate the first needle 12 and the second needle 13. As shown, the electrode lead 3 is received in the second needle 13, and the second needle 13 includes a slot 18 for connecting to the implant housing 2 in the first needle 12. The slot 18 extends partially along the second needle 13 from the tip 15. The slot 18 may extend the full length of the second needle 13. The second needle 13 may be in the form of a sheath. The first needle 12 receives the implant housing 2.

The first needle 12 includes a bevel tip 14, and the second needle include a bevel tip 15. The bevel tips 14, 15 are sharp for piercing a patient's skin and penetrating the tissue during use.

As shown in FIG. 7, the second needle 13 extends through the lumen of the first needle 12. In particular, as shown in FIG. 8B the first needle 12 includes a primary portion 19A and a secondary portion 19B. The primary and secondary portions 19A, 19B are merged such that the first needle 12 has a single lumen. The primary and secondary portions 19A, 19B are shaped to define the two distinct portions 19A, 19B.

The primary portion 19A is shaped to receive the implant housing 2. In particular, the primary portion 19A is sized to receive the implant housing 2 and has a substantially circular cross-section that retains the implant housing 2 in axial alignment within the primary portion 19A.

The secondary portion 19B is shaped to receive the second needle 13. In particular, the secondary portion 19B is sized to receive the second needle 13 and has a substantially circular cross-section that retains the second needle 13 in axial alignment within the secondary portion 19B.

Accordingly, the first needle 12 is shaped to receive the implant housing 2 and the second needle 13, and to permit the second needle 13 to slide towards the retracted position. The position of the second needle 13 within the lumen of the first needle 1 beneficially means that there is only one puncture wound formed in the patient's skin, as the first needle 12 will enlarge the puncture wound formed by the second needle 13 during use.

In alternative examples the second needle 13 does not pass into, or through, the lumen of the first needle 12. Instead, the second needle 13 can extend through another part of the handle 11.

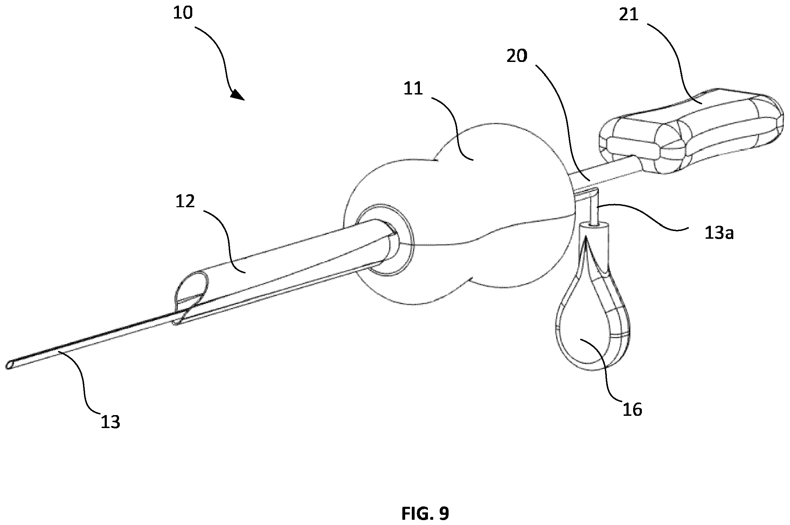

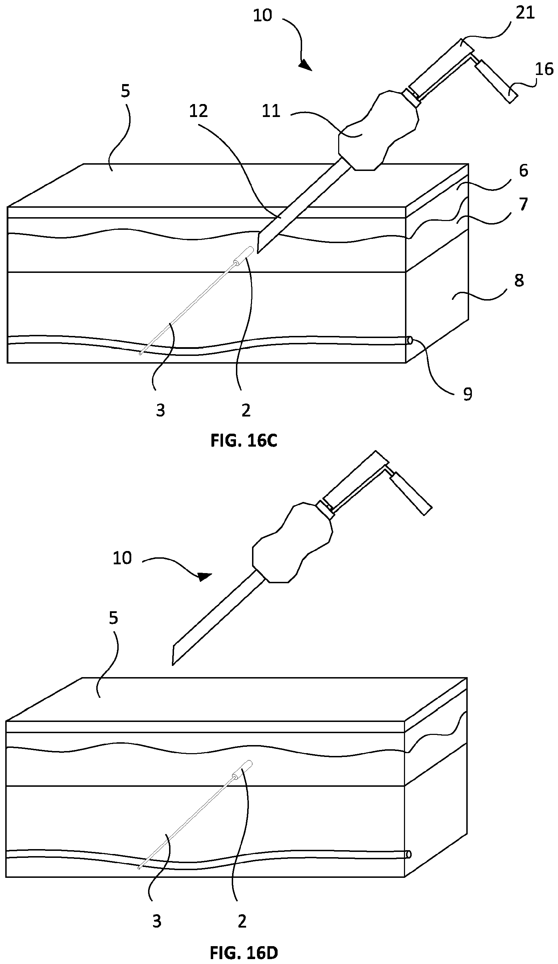

FIGS. 9 to 11 illustrate an example implant delivery device 10. As illustrated, the implant delivery device 10 includes a handle 11, a first needle 12 and a second needle 13 in the configuration as described above with reference to FIGS. 3 to 8B. In particular, the first needle 12 is fixed to the handle 11 and the second needle 13 is retractable from an extended position (shown in FIG. 9) where it extends past the end of the first needle 12, and a retracted position (see FIG. 16C) where the second needle 13 is retracted towards the handle 11 to release the electrode lead (3, see FIG. 3).

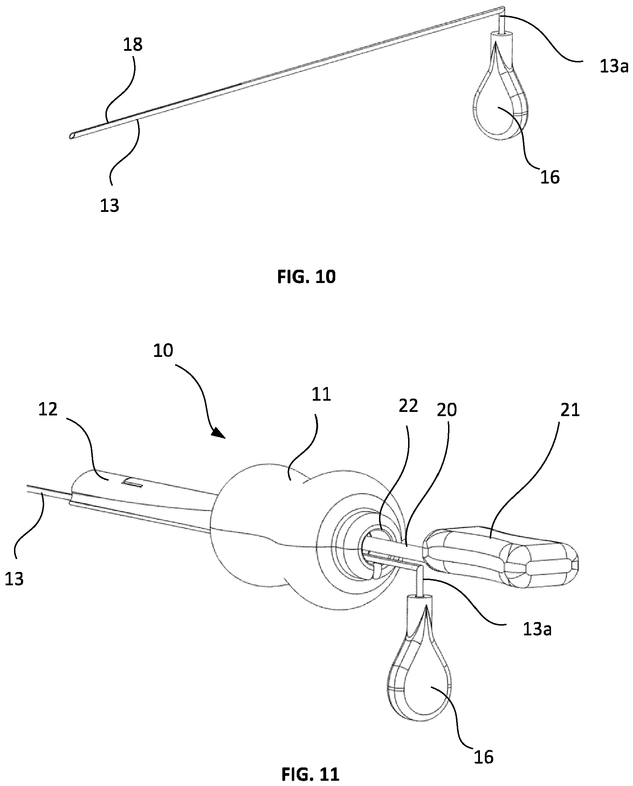

As shown in FIGS. 9 and 10, the second needle 13 extends through the handle 11 and a portion 13a of the second needle 13 is attached to the actuation tab 16.

Also shown in FIG. 9 is an implant deployment member 20, which includes a gripping tab 21. As explained further hereinafter, the implant deployment member 20 may be in the form of a tube or plunger arranged to slide into the first needle 12 and push the implant housing 2 out of the first needle 12.

As shown in FIG. 11, the handle 11 includes an opening 22 in which the first needle 12 is mounted, and through which the second needle 13 and the implant deployment member 20 pass.

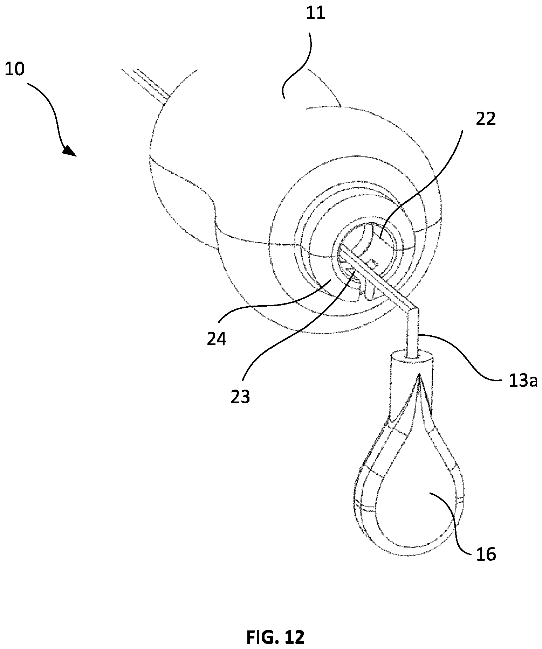

FIG. 12 shows an example of the locking mechanism 17 described with reference to FIGS. 4 to 6. The locking mechanism is arranged to releasably lock the position of the second needle 13 relative to the handle 11 and first needle 12. In this example, the locking mechanism comprises a locking slot 23 formed in a locking ring 24 on the handle 11. The locking ring 24 may be an integral part of the handle 11 or attached to the handle 11. The locking slot 23 may be an L-slot or a J-slot shaped to receive a part of the second needle 13, in particular the portion 13a of the second needle 13 that the actuation tab 16 is attached to. The portion 13a can enter the slot and rotation of the second needle 13 (by the actuation tab 16) moves the portion 13a into an end of the locking slot 23 that restrains axial movement of the second needle 13. Accordingly, the second needle 13 can be moved between a locked position and an unlocked position by rotating the second needle 13, in particular the actuation tab 16, to move the portion 13a into and out of the locking slot 23.

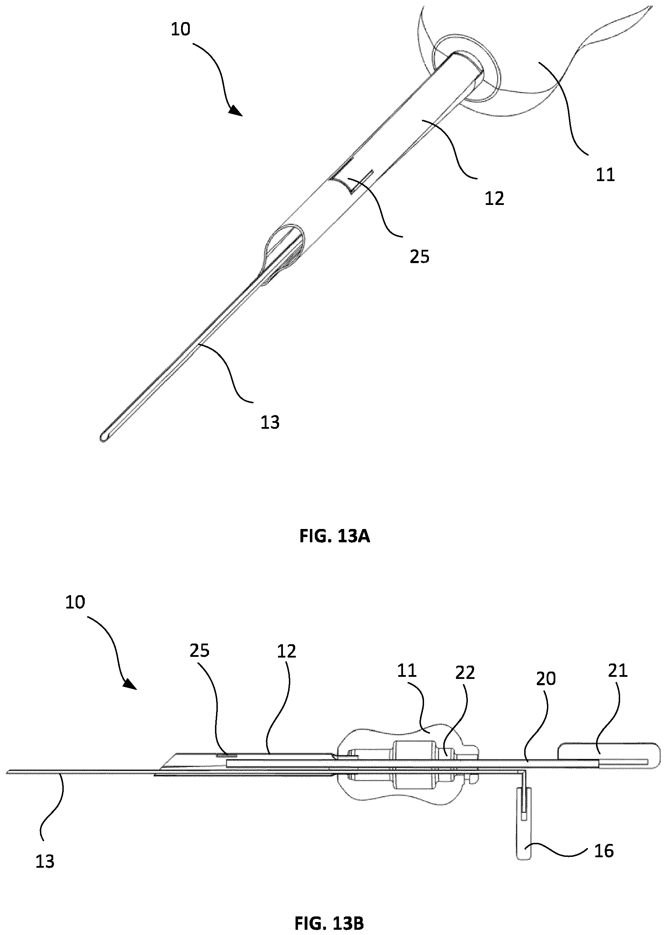

FIGS. 13A and 13B illustrate an example of a retaining member configured to releasably secure the implant housing 2 in the first needle 12. As shown, in this example the first needle 12 comprises a retaining member in the form of a retaining tab 25 arranged to contact the implant housing (2, see FIG. 7) and retain the implant housing in the first needle 12. The retaining tag 25 is formed by a U-shaped cut in the first needle 12 that forms the retaining tab 25, which is partially bent inwards to narrow the internal diameter of the first needle 12 at the location of the retaining tab 25.

In some examples, the implant housing 2 may comprise a recess arranged to correspond with the retaining tab 25. In other examples, the retaining tab 25 contacts a side wall of the implant housing 2.

The retaining tab 25 may be configured such that the implant housing 2 can be pulled from the first needle 12 with a relatively low force to deploy the implant housing 2 from the first needle 12. Accordingly, friction between the electrode lead 3 and tissue of the patient may provide sufficient holding force for the retaining force of the retaining tab 25 to be overcome by pulling the implant delivery device 10 from the patient after retracting the second needle 13, as described above.

In another example, the implant delivery device 10 further comprises an implant deployment member 20 as shown in FIG. 13B. The implant deployment member 20 extend through the opening 22 in the handle and into the first needle 12. From here, the implant deployment member 20 can function as a plunger to push the implant housing 2 out of the first needle 12. The implant deployment member 20 is provided with a gripping tab 21 for actuation of the implant deployment member 20.

In examples, the implant deployment member 20 may comprise a locking tab arranged to prevent axial movement of the implant deployment member 20 towards the implant housing 2 until the locking tab is disengaged, for example by rotation of the implant deployment member 20 or gripping tab 21.

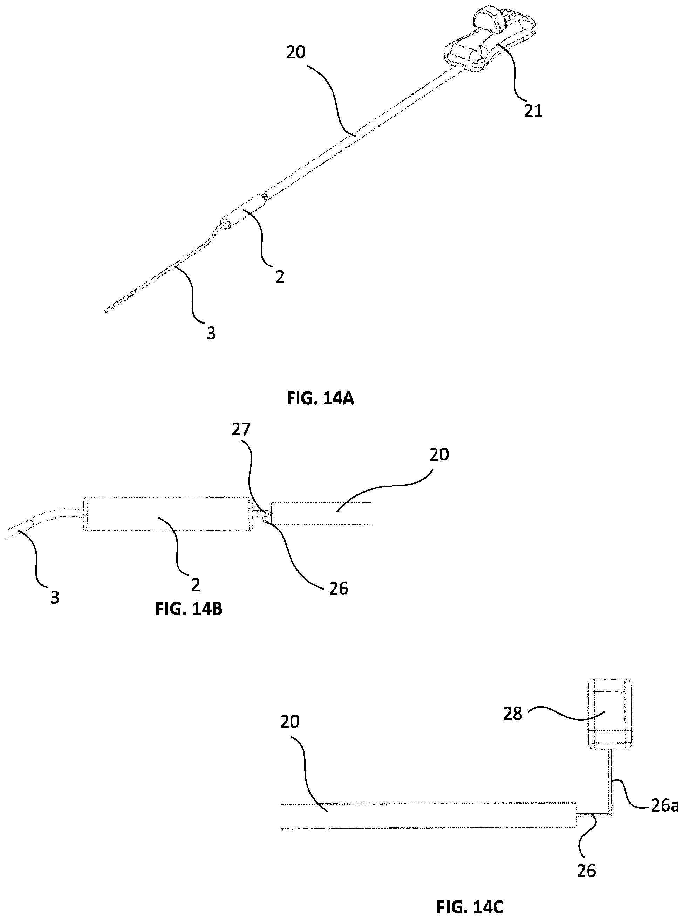

FIGS. 14A to 14C illustrate another example of a retaining member configured to releasably secure the implant housing 2 in the first needle 12. In this example, the retaining member comprises a shape memory member 26 that extends through the handle 11, and in particular through the implant deployment member 20, between the gripping tab 21 and the implant housing 2.

As shown in FIG. 14B, the implant housing 2 comprises an attachment point 27, for example a hole or hook, that an end of the shape memory member 26 hooks onto to hold the implant housing 2 in the first needle 12.

As shown in FIG. 14C an opposite end 26a of the shape memory member 26 to the hook 27 is attached to an actuation tab 28 that is slidably mounted to the gripping tab 21. Accordingly, moving the actuation tab 28 relative to the gripping tab 21 pulls on shape memory member 26. Pulling the actuation tab 28 can thereby deform the hook at the end of the shape memory member 26 and disengage it from the attachment point 27, thereby releasing the implant housing 2.

In examples the shape memory member 26 comprises a shape memory polymer or a shape memory alloy such as a Nitinol bar. In other examples the shaper memory member 26 may comprise a bi-metallic strip, spring wire, or piano wire. The shape memory member 26 has a hooked end that engages the attachment point 27 on the implant housing 2. The hooked end can be formed as shape memory and act to prevent the implant housing 2 from moving out of the first needle 12 until the actuation tab 28 has been pulled to disengage the hooked end from the attachment point 27.

The actuation tab 28 can be releasably locked relative to the gripping tab 21 to prevent unintentional release of the implant housing 2 from the first needle 12.

Once the shape memory member 26 has been disengaged from the attachment point 27 the implant housing 2 may be pulled out of the first needle 12 by friction between the electrode lead 3 and the tissue of the patient, and/or an implant deployment member 20 may act as a plunger as described previously.

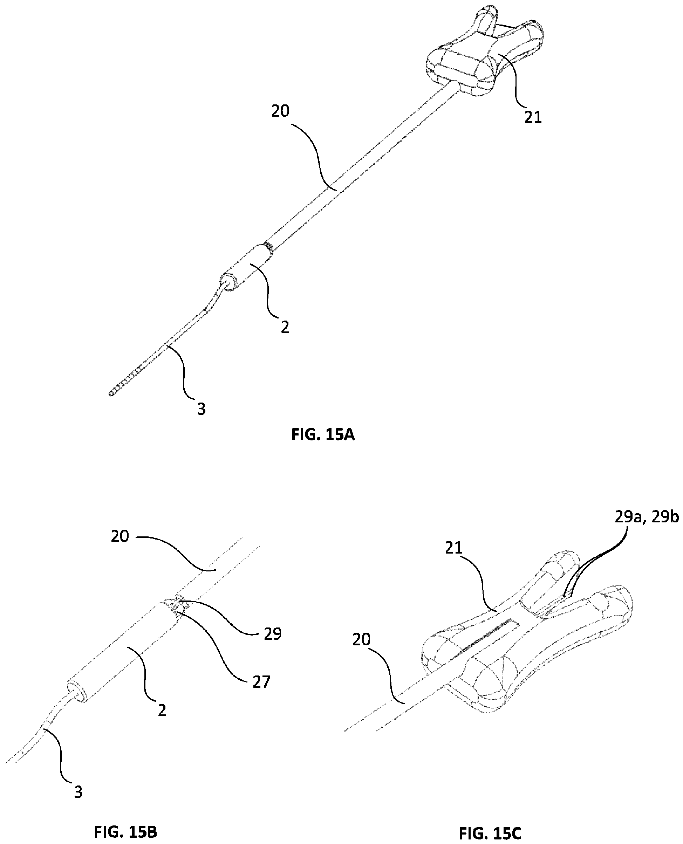

FIGS. 15A to 15C illustrate another example of a retaining member configured to releasably secure the implant housing 2 in the first needle 12. In this example the retaining member comprises a thread 29 extending from the gripping tab 21 to the implant housing 2 in the first needle 12. The thread 29 is looped through the attachment point 27 on the implant housing 2 and both ends 29a, 29b are secured at the gripping tab 21. For example, the ends 29a, 29b may be tied to each other, or to a part of the gripping tab 21. Preferably, the ends 29a, 29b are secured to the gripping tab 21 such that the ends 29a, 29b can be readily released or such that a part of the thread 29 can be easily accessed to be cut.

Accordingly, the thread 29 acts to hold the implant housing 2 in the first needle 12 until the thread 29 is cut or untied, allowing the thread 29 to be removed.

Once the implant housing 2 has been released the implant housing 2 may be pulled out of the first needle 12 by friction between the electrode lead 3 and the tissue of the patient, and/or an implant deployment member 20 may act as a plunger as described previously.

The thread may be a Kevlar thread.

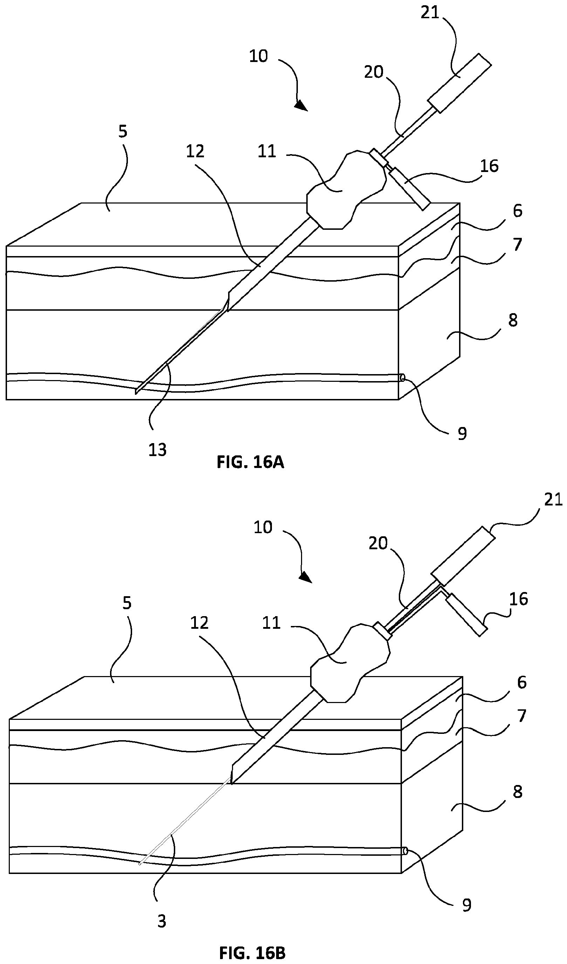

FIGS. 16A to 16 D illustrate a method of operating the implant deployment device 10 to deploy the medical implant 1 into a patient.

In these examples the implant delivery device 10 comprises a handle 11, a first needle 12 with the implant housing 2, and a second needle with the electrode lead 3 as described above. The implant delivery device 10 also includes an actuation tab 16 for retracting the second needle 13, and an implant deployment member 20 for pushing the implant housing 2 out of the first needle 12 as described above.

The implant delivery device 10 of FIGS. 16A to 16D may include a retaining member as described in any of FIGS. 13A to 15C.

FIG. 16A shows the implant delivery device 10 in an initial state, with the second needle 13 in an extended position covering the electrode lead (not shown in FIG. 16A). The second needle 13 is locked to the handle 11 by a locking mechanism, for example the locking slot 23 described with reference to FIG. 12.

The implant delivery device 10 and medical implant 1 are pre-assembled and packaged as a single-use unit. Accordingly, the operator is provided with an implant delivery device 10 with a medical implant 1 already loaded into the first and second needles 12, 13.

In this state an operator pushes the first and second needles 12, 13 through the patient's skin 5 and into the position for deploying the medical implant 1. The operator may use an ultrasound imaging device to help guide the second needle 13 towards the nerve 9.

In the appropriate position, shown in FIG. 16A, the second needle 16 is in the muscle tissue 8, proximal to the nerve 9, and the end of first needle 12 that houses the implant housing 2 is positioned in the subcutaneous tissue 7. Both the first and second needles 12, 13 have passed through the epidermis 5 and dermis 6. As the second needle 13 is arranged within the lumen of the first needle 12 there is only a single puncture through the epidermis 5 and dermis 6.

In one example, the second needle 13 may comprise one or more side openings corresponding to the electrodes (4, see FIG. 1) on the electrode lead, which permits the position of the electrode lead to be tested before retracting the second needle 13. If such testing shows that the electrode lead is not in the correct location relative to the nerve 9 then the implant delivery device 10 can be at least partially removed and the second needle 13 can be repositioned.

As shown in FIG. 16B, next the operator unlocks the second needle 13 (e.g., rotates the second needle 13 to disengage the locking mechanism of FIG. 12), and retracts the actuation tab 16 away from the patient. This moves the second needle 13 into the retracted position within the first needle 12 and releases the electrode lead 3.

In one example, the second needle 13 is only partially retracted in order to expose only an end of the electrode lead 3, where the electrodes (4, see FIG. 1) are located. This partial retraction permits testing of the position of the electrode lead 3 relative to the nerve 9. If such testing shows that the electrode lead is not in the correct location relative to the nerve 9 then the second needle 13 can be pushed back out again to re-sheath the electrode lead 3, and the implant delivery device 10 can be at least partially removed and the second needle 13 can be repositioned.

Once the position of the electrode lead 3 is correct, the second needle 13 is fully retracted to release the electrode lead 3.

Next, as shown in FIG. 16C, the implant housing 2 is released and deployed from the first needle 12. In particular, if the implant delivery device 10 includes a retaining member as described in any of FIGS. 13A to 15C, then the retaining member is released to release the implant housing 2.

In some examples, friction between the electrode lead 3 and the tissue, in particular the muscle 8, provides a holding force sufficient to pull the implant housing 2 out of the first needle 12 when the implant delivery device 10 is withdrawn from the patient.

In other examples, as illustrated in FIG. 16C, the implant deployment member 20 (and gripping tab 21) are pushed towards the handle 11 to push the implant housing 2 out of the first needle 12. The implant deployment member 20 may be pushed towards the handle 11 while simultaneously moving the handle 11 away from the skin 5 to deploy the implant housing 2 without pulling or pushing on the electrode lead 3.

Accordingly, the medical implant 1 is deployed, as shown in FIG. 16D, and the implant delivery device 10 can be removed from the patient.

In summary, there is provided an implant delivery device for implanting a medical implant in a patient, the medical implant comprising an implant housing and an elongate electrode lead extending from the implant housing. The implant delivery device comprises a handle, a first needle fixed to the handle and extending in a longitudinal direction, and a second needle having a higher gauge than the first needle. The first needle has a lumen adapted to receive the implant housing of the medical implant, and the second needle has a lumen adapted to receive the elongate electrode lead of the medical implant. The second needle is retractably mounted to the handle such that the second needle is movable between an extended position and a retracted position. In the extended position the second needle extends beyond the first needle in the longitudinal direction such that during use the first needle and the second needle can simultaneously position the implant housing at a first depth and the electrode lead at a second depth greater than the first depth. In the retracted position the second needle is retracted towards the handle to release the electrode lead from the second needle.

There is also provided a method of implanting a medical implant 1 using the implant delivery device 10. The method includes positioning the first and second needles 12, 13 in the patient to position the medical implant 1 relative to the nerve 9, retracting the second needle 13 to release the electrode lead 3, and then deploying the implant housing 2 and removing the implant delivery device 10, optionally simultaneously.

In some examples the method may include releasing a retaining member to release the implant housing 2 from the first needle 12. In examples, the method may comprise unlocking the second needle 13 from the handle 11 before retracting the second needle 13. In examples, the method may include partially retracting the second needle 13, testing the position of the electrode lead 3 relative to the nerve 9, then re-sheathing the electrode lead 3 and repositioning, if required, before retracting the second needle 13 and deploying the medical implant 1.

Throughout the description and claims of this specification, the words "comprise" and "contain" and variations of them mean "including but not limited to", and they are not intended to (and do not) exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

The present invention is set out in the appended claims. The embodiments, examples or aspects according to the present description which do not fall within the scope of said claims are provided for illustrative purposes only and do not form part of the present invention.

## Claims

1. An implant delivery device (10) for implanting a medical implant (1) in a patient, the medical implant comprising an implant housing (2) and an elongate electrode lead (3) extending from the implant housing, and wherein the implant delivery device comprises:
a handle (11),
a first needle (12) fixed to the handle and extending in a longitudinal direction, the first needle comprising a lumen adapted to receive the implant housing of the medical implant, and
a second needle (13) having a higher gauge than the first needle, the second needle comprising a lumen adapted to receive the elongate electrode lead of the medical implant, and wherein the second needle is retractably mounted to the handle such that the second needle is movable between:
an extended position in which the second needle extends beyond the first needle in the longitudinal direction such that during use the first needle and the second needle can simultaneously position the implant housing at a first depth and the electrode lead at a second depth greater than the first depth, and
a retracted position in which the second needle is retracted towards the handle to release the electrode lead from the second needle.

2. The implant delivery device of claim 1, further comprising a retaining member (25, 26, 29) adapted to releasably secure the implant housing in the lumen of the first needle.

3. The implant delivery device of claim 2, wherein the retaining member comprises a retaining tab (25) formed in a wall of the first needle and adapted to engage the implant housing in the lumen of the first needle.

4. The implant delivery device of claim 2, wherein the implant housing comprises an attachment point, for example a hole (27), and the retaining member (26, 29) extends from a part of the handle to the attachment point of the implant housing, the retaining member being detachable to release the implant housing.

5. The implant delivery device of claim 4, wherein the retaining member (29) comprises a thread that is looped through the attachment point of the implant housing, and wherein the thread can be split, for example cut or untied, to release the implant housing.

6. The implant delivery device of claim 4, wherein the retaining member (26) comprises a shape memory member having a hooked end adapted to engage the attachment point of the implant housing, and wherein an opposite end (26a) of the shape memory housing is arranged to be moved to deform the hooked end to release the implant housing.

7. The implant delivery device of any preceding claim, wherein the second needle comprises an actuation tab (16) arranged adjacent to the handle, the actuation tab being configured for actuation by a user to move the second needle into the retracted position.

8. The implant delivery device of claim 7, further comprising a locking mechanism (17) adapted to lock the second needle in the extended position.

9. The implant delivery device of claim 8, wherein the locking mechanism comprises a locking slot (23) in the handle, and wherein the second needle is adapted to engage the locking slot such that in one rotational position of the second needle relative to the handle the second needle is locked in the axial direction relative to the handle, and in another rotational position of the second needle relative to the housing the second needle is movable relative to the handle in an axial direction.

10. The implant delivery device of any preceding claim, further comprising an implant deployment member (20) comprising a pusher extending from adjacent to the handle into the lumen of the first needle, the implant deployment member being slidable relative to the first needle to push the implant housing out of the first needle.

11. The implant delivery device of any preceding claim, wherein the second needle comprises a slot (18) extending along one side and extending into the lumen.

12. The implant delivery device of claim 11, wherein the first needle and the second needle are non-concentrically arranged such that a portion of the electrode lead extends from the lumen of the second needle through the slot to the implant housing during use.

13. The implant delivery device of claim 12, wherein the second needle is at least partly disposed in the lumen of the first needle.

14. The implant delivery device of claim 13, wherein the lumen of the first needle comprises a primary portion (19A) adapted to receive the implant housing and a secondary portion (19B) adapted to receive the second needle, the primary portion and the secondary portion each comprising a partially circular cross-section.

15. The implant delivery device of any preceding claim further comprising a medical implant comprising an implant housing disposed in the lumen of the first needle and an electrode lead extending from the implant housing into the lumen of the second needle.

## Patentansprüche

1. Implantatzufuhrvorrichtung (10) zum Implantieren eines medizinischen Implantats (1) in einen Patienten, das medizinische Implantat umfassend ein Implantatgehäuse (2) und eine längliche Elektrodenleitung (3), die sich von dem Implantatgehäuse erstreckt, und wobei die Implantatzufuhrvorrichtung Folgendes umfasst:
einen Haltegriff (11),
eine erste Nadel (12), die am Haltegriff befestigt ist und sich in Längsrichtung erstreckt, die erste Nadel umfassend ein Lumen, das angepasst ist, um das Implantatgehäuse des medizinischen Implantats aufzunehmen, und
eine zweite Nadel (13), die eine höhere Stärke als die erste Nadel aufweist, wobei die zweite Nadel ein Lumen umfasst, das angepasst ist, um die längliche Elektrodenleitung des medizinischen Implantats aufzunehmen, und wobei die zweite Nadel zurückziehbar an dem Haltegriff montiert ist, sodass die zweite Nadel beweglich ist zwischen:
einer ausgefahrenen Position, in der sich die zweite Nadel in Längsrichtung über die erste Nadel hinaus erstreckt, sodass im Gebrauch die erste Nadel und die zweite Nadel gleichzeitig das Implantatgehäuse in einer ersten Tiefe und die Elektrodenleitung in einer zweiten Tiefe, die größer ist als die erste Tiefe, positionieren können, und
einer zurückgezogenen Position, in der die zweite Nadel zu dem Haltegriff zurückgezogen ist, um die Elektrodenleitung von der zweiten Nadel freizugeben.

2. Implantatzufuhrvorrichtung nach Anspruch 1, ferner umfassend ein Halteelement (25, 26, 29), das angepasst ist, um das Implantatgehäuse in dem Lumen der ersten Nadel lösbar zu sichern.

3. Implantatzufuhrvorrichtung nach Anspruch 2, wobei das Halteelement eine Haltelasche (25) umfasst, die in einer Wand der ersten Nadel gebildet und angepasst ist, um das Implantatgehäuse in dem Lumen der ersten Nadel einzugreifen.

4. Implantatzufuhrvorrichtung nach Anspruch 2, wobei das Implantatgehäuse einen Anbringungspunkt, zum Beispiel ein Loch (27), umfasst und das Halteelement (26, 29) sich von einem Teil des Haltegriff zu dem Befestigungspunkt des Implantatgehäuses erstreckt, wobei das Halteelement ablösbar ist, um das Implantatgehäuse freizugeben.

5. Implantatzufuhrvorrichtung nach Anspruch 4, wobei das Halteelement (29) einen Faden umfasst, der durch den Befestigungspunkt des Implantatgehäuses geschlungen ist, und wobei der Faden geteilt, beispielsweise geschnitten oder freigegeben werden kann, um das Implantatgehäuse freizugeben.

6. Implantatzufuhrvorrichtung nach Anspruch 4, wobei das Halteelement (26) ein Formgedächtniselement umfasst, das ein hakenförmiges Ende aufweist, das angepasst ist, um den Befestigungspunkt des Implantatgehäuses einzugreifen, und wobei ein gegenüberliegendes Ende (26a) des Formgedächtnisgehäuses angeordnet ist, um bewegt zu werden, um das hakenförmige Ende zu verformen, um das Implantatgehäuse freizugeben.

7. Implantatzufuhrvorrichtung nach einem der vorherigen Ansprüche, wobei die zweite Nadel eine Betätigungslasche (16) umfasst, die neben dem Haltegriff angeordnet ist, wobei die Betätigungslasche für eine Betätigung durch einen Benutzer konfiguriert ist, um die zweite Nadel in die zurückgezogene Position zu bewegen.

8. Implantatzufuhrvorrichtung nach Anspruch 7, die ferner umfassend einen Verriegelungsmechanismus (17), der angepasst ist, um die zweite Nadel in der ausgefahrenen Position zu verriegeln.

9. Implantatzufuhrvorrichtung nach Anspruch 8, wobei der Verriegelungsmechanismus einen Verriegelungsschlitz (23) in dem Haltegriff umfasst, und wobei die zweite Nadel angepasst ist, um den Verriegelungsschlitz einzugreifen, sodass die zweite Nadel in einer Drehposition der zweiten Nadel in Bezug auf den Haltegriff in der axialen Richtung in Bezug auf den Haltegriff verriegelt ist, und in einer anderen Drehposition der zweiten Nadel in Bezug auf das Gehäuse die zweite Nadel in Bezug auf den Haltegriff in einer axialen Richtung beweglich ist.

10. Implantatzufuhrvorrichtung nach einem der vorherigen Ansprüche, ferner umfassend ein Implantatentfaltungselement (20), umfassend einen Schieber, der sich von der Nähe des Haltegriffs in das Lumen der ersten Nadel erstreckt, wobei das Implantatentfaltungselement in Bezug auf die erste Nadel verschiebbar ist, um das Implantatgehäuse aus der ersten Nadel herauszuschieben.

11. Implantatzufuhrvorrichtung nach einem der vorherigen Ansprüche, wobei die zweite Nadel einen Schlitz (18) umfasst, der sich entlang einer Seite erstreckt und sich in das Lumen erstreckt.

12. Implantatzufuhrvorrichtung nach Anspruch 11, wobei die erste Nadel und die zweite Nadel nicht-konzentrisch angeordnet sind, sodass sich ein Abschnitt der Elektrodenleitung im Gebrauch von dem Lumen der zweiten Nadel durch den Schlitz zu dem Implantatgehäuse erstreckt.

13. Implantatzufuhrvorrichtung nach Anspruch 12, wobei die zweite Nadel zumindest teilweise in dem Lumen der ersten Nadel angeordnet ist.

14. Implantatzufuhrvorrichtung nach Anspruch 13, wobei das Lumen der ersten Nadel einen primären Abschnitt (19A), der angepasst ist, um das Implantatgehäuses aufzunehmen, und einen sekundären Abschnitt (19B), der angepasst ist, um die zweite Nadel aufzunehmen, umfasst, der primäre Abschnitt und der sekundäre Abschnitt jeweils umfassend einen teilweise kreisförmigen Querschnitt.

15. Implantatzufuhrvorrichtung nach einem der vorherigen Ansprüche, ferner umfassend ein medizinisches Implantat, umfassend ein in dem Lumen der ersten Nadel angeordnetes Implantatgehäuse und eine Elektrodenleitung, die sich von dem Implantatgehäuse in das Lumen der zweiten Nadel erstreckt.

## Revendications

1. Dispositif de pose d'implant (10) permettant l'implantation d'un implant médical (1) chez un patient, l'implant médical comprenant un boîtier d'implant (2) et un fil d'électrode allongé (3) s'étendant depuis le boîtier d'implant, et ledit dispositif de pose d'implant comprenant :
une poignée (11),
une première aiguille (12) fixée à la poignée et s'étendant suivant une direction longitudinale, la première aiguille comprenant une lumière adaptée à la réception du boîtier d'implant de l'implant médical, et
une seconde aiguille (13) comportant un calibre plus élevé que la première aiguille, la seconde aiguille comprenant une lumière adaptée à la réception du fil d'électrode allongé de l'implant médical, et ladite seconde aiguille étant montée de manière rétractable sur la poignée de sorte que la seconde aiguille soit mobile entre :
une position déployée dans laquelle la seconde aiguille s'étend au-delà de la première aiguille suivant la direction longitudinale de sorte que, lors de l'utilisation, la première aiguille et la seconde aiguille puissent positionner simultanément le boîtier d'implant à une première profondeur et le fil d'électrode à une seconde profondeur supérieure à la première profondeur, et
une position rétractée dans laquelle la seconde aiguille est rétractée en direction de la poignée pour libérer le fil d'électrode de la seconde aiguille.

2. Dispositif de pose d'implant selon la revendication 1, comprenant en outre un élément de retenue (25, 26, 29) adapté à la fixation de manière amovible du boîtier d'implant dans la lumière de la première aiguille.

3. Dispositif de pose d'implant selon la revendication 2, ledit élément de retenue comprenant une languette de retenue (25) formée dans une paroi de la première aiguille et adaptée à l'engagement du boîtier d'implant dans la lumière de la première aiguille.

4. Dispositif de pose d'implant selon la revendication 2, ledit boîtier d'implant comprenant un point de fixation, par exemple un trou (27), et ledit élément de retenue (26, 29) s'étendant depuis une partie de la poignée jusqu'au point de fixation du boîtier d'implant, ledit élément de retenue étant détachable pour libérer le boîtier d'implant.

5. Dispositif de pose d'implant selon la revendication 4, ledit élément de retenue (29) comprenant un fil qui forme une boucle traversant le point de fixation du boîtier d'implant, et ledit fil pouvant être fendu, par exemple coupé ou détaché, pour libérer le boîtier d'implant.

6. Dispositif de pose d'implant selon la revendication 4, ledit élément de retenue (26) comprenant un élément à mémoire de forme comportant une extrémité recourbée adaptée à l'engagement du point de fixation du boîtier d'implant, et une extrémité opposée (26a) du boîtier à mémoire de forme étant destinée à se déplacer afin de déformer l'extrémité recourbée pour libérer le boîtier d'implant.

7. Dispositif de pose d'implant selon l'une quelconque des revendications précédentes, ladite seconde aiguille comprenant une patte d'actionnement (16) disposée adjacente à la poignée, la patte d'actionnement étant conçue pour être actionnée par un utilisateur pour déplacer la seconde aiguille dans la position rétractée.

8. Dispositif de pose d'implant selon la revendication 7, comprenant en outre un mécanisme de verrouillage (17) adapté au verrouillage de la seconde aiguille dans la position déployée.

9. Dispositif de pose d'implant selon la revendication 8, ledit mécanisme de verrouillage comprenant une fente de verrouillage (23) dans la poignée, et ladite seconde aiguille étant adaptée à l'engagement dans la fente de verrouillage de sorte que, dans une position de rotation de la seconde aiguille par rapport à la poignée, la seconde aiguille soit bloquée dans la direction axiale par rapport à la poignée, et, dans une autre position de rotation de la seconde aiguille par rapport au boîtier, la seconde aiguille soit mobile par rapport à la poignée dans une direction axiale.

10. Dispositif de pose d'implant selon l'une quelconque des revendications précédentes, comprenant en outre un élément de déploiement d'implant (20) comprenant un poussoir s'étendant d'une position adjacente à la poignée dans la lumière de la première aiguille, l'élément de déploiement d'implant pouvant coulisser par rapport à la première aiguille pour pousser le boîtier d'implant hors de la première aiguille.

11. Dispositif de pose d'implant selon l'une quelconque des revendications précédentes, ladite seconde aiguille comprenant une fente (18) s'étendant le long d'un côté et s'étendant dans la lumière.

12. Dispositif de pose d'implant selon la revendication 11, ladite première aiguille et ladite seconde aiguille étant disposées de manière non concentrique de sorte qu'une partie du fil d'électrode s'étende depuis la lumière de la seconde aiguille à travers la fente jusqu'au boîtier d'implant pendant l'utilisation.

13. Dispositif de pose d'implant selon la revendication 12, ladite seconde aiguille étant au moins partiellement disposée dans la lumière de la première aiguille.

14. Dispositif de pose d'implant selon la revendication 13, ladite lumière de la première aiguille comprenant une partie primaire (19A) adaptée à la réception du boîtier d'implant et une partie secondaire (19B) adaptée à la réception de la seconde aiguille, la partie primaire et la partie secondaire comprenant chacune une section transversale partiellement circulaire.

15. Dispositif de pose d'implant selon l'une quelconque des revendications précédentes, comprenant en outre un implant médical comprenant un boîtier d'implant disposé dans la lumière de la première aiguille et un fil d'électrode s'étendant depuis le boîtier d'implant dans la lumière de la seconde aiguille.
